# FASCICULE DE BREVET EUROPEEN

(11) **EP 3 451 941 B1**
(45) Date de publication et mention de la délivrance du brevet: **21.10.2020**
(21) Numéro de dépôt: 17723948.0
(22) Date de dépôt: 28.04.2017
(51) Int. Cl.: A61B 17/12, A61B 17/42, A61M 25/10

(54) **BALLONNET GONFLABLE ET DÉTACHABLE, DESTINÉ À ÊTRE IMPLANTÉ DANS UNE CAVITÉ CORPORELLE ET NÉCESSAIRE DE TRAITEMENT**
AUFBLASBARER UND LÖSBARER BALLON ZUR IMPLANTATION IN EINE KÖRPERHÖHLE UND ZUGEHÖRIGES BEHANDLUNGSKIT
INFLATABLE AND DETACHABLE BALLOON INTENDED TO BE IMPLANTED IN A BODY CAVITY AND ASSOCIATED TREATMENT KIT

(30) Priorité: 02.05.2016 FR 1653954
(43) Date de publication de la demande: 13.03.2019
(73) Titulaire: Université de Strasbourg, 67000 Strasbourg (FR); Institut National de la Santé et de la Recherche Médicale (INSERM), 75013 Paris (FR); Hôpitaux Universitaires de Strasbourg (HUS), 67000 Strasbourg (FR); Institut Hospitalo-Universitaire De Strasbourg, 67000 Strasbourg (FR); Institut De Recherche Contre Les Cancers De L'Appareil Digestif, 67091 Strasbourg (FR); BS Medical Tech Industry, 67470 Niederroedern (FR)
(72) Inventeur: SANANES, Nicolas, 67000 Strasbourg (FR); FAVRE, Romain, 67000 Strasbourg (FR); DEBRY, Christian, 67000 Strasbourg (FR); GOETZ, Christian, 67600 Sélestat (FR); MUTET, Bruno, 67100 Strasbourg (FR); HERNANDEZ, Juan, 67000 Strasbourg (FR); BASCH, Bertrand, 67620 Soufflenheim (FR); BASCH, Raymond, 67460 Mothern (FR); LEROY, Joël, 67300 Schiltigheim (FR)
(74) Mandataire: Lavoix
(86) Numéro de dépôt international: PCT/EP2017/060282
(87) Numéro de publication internationale: WO 2017/191069

(56) Documents cités:
- WO-A1-2013/146210
- US-A1- 2010 241 241
- US-A1- 2012 184 808
- J. DEPREST ET AL: "Fetoscopic tracheal occlusion(FETO) for severe congenital diaphragmatic hernia: evolution of a technique and preliminary results", ULTRASOUND IN OBSTETRICS AND GYNECOLOGY, vol. 24, no. 2, 27 juillet 2004 (2004-07-27), pages 121-126, XP055255866, GB ISSN: 0960-7692, DOI: 10.1002/uog.1711
- DEPREST J ET AL: "Technical aspects of fetal endoscopic tracheal occlusion for congenital diaphragmatic hernia", JOURNAL OF PEDIATRIC SURGERY, W. B. SAUNDERS COMPANY, US, vol. 46, no. 1, 1 janvier 2011 (2011-01-01), pages 22-32, XP027592714, ISSN: 0022-3468, DOI: 10.1016/J.JPEDSURG.2010.10.008 [extrait le 2011-01-01]
- THE FETO TASK GROUP DEPREST J ET AL: "Fetal Intervention for Congenital Diaphragmatic Hernia: The European Experience", SEMINARS IN PERINATOLOGY, W.B. SAUNDERS, GB, vol. 29, no. 2, 1 avril 2005 (2005-04-01), pages 94-103, XP004937626, ISSN: 0146-0005, DOI: 10.1053/J.SEMPERI.2005.04.006

## Description

La présente invention concerne un ballonnet gonflable, destiné à être implanté dans une cavité corporelle, comportant :
- une poche formée d'une paroi étanche délimitant un espace intérieur ;
- une vanne de remplissage de l'espace intérieur par un fluide, propre à être obturée après remplissage de l'espace intérieur.

Le document US 2010/241241 A1 décrit un ballonnet gonflable. Le dégonflage du ballon est réalisé à l'aide d'une valve électromagnétique.

Le ballonnet est propre à être inséré à l'extrémité d'un dispositif de largage et de gonflage et à être détaché du dispositif.

Un tel ballonnet est destiné notamment à être implanté dans la trachée d'un fœtus pour réaliser une occlusion trachéale fœtale lorsque le fœtus est atteint de hernie diaphragmatique congénitale. Le ballonnet peut également être utilisé dans le cadre d'indications potentielles, comme la rupture prématurée des membranes ou toute autre affection associée à une hypoplasie pulmonaire fœtale. La hernie diaphragmatique congénitale est une maladie affectant sporadiquement les fœtus, avec une incidence généralement comprise entre 1/3000 et 1/5000 parmi les nouveau-nés.

Cette hernie se traduit par une invasion d'organes de l'abdomen, tels que l'intestin, l'estomac et/ou le foie dans la cavité thoracique en raison du défaut diaphragmatique. Ceci applique une pression sur les poumons en développement et provoque une hypoplasie pulmonaire susceptible d'engendrer une insuffisance respiratoire, voire parfois la mort du nouveau-né. La mortalité actuelle résultant d'une hernie diaphragmatique congénitale isolée est estimée à 30 % environ par certaines études. L'hypoplasie pulmonaire est plus ou moins sévère en fonction de l'importance de la hernie. Les conséquences une fois l'enfant né sont l'insuffisance respiratoire, mais aussi l'hypertension artérielle pulmonaire.

Pour pallier ce problème, il est connu notamment de l'article « Technical Aspects of Fetal Endoscopie Tracheal Occlusion for Congenital Diaphragmatic Hernia », Journal of Pediatric Surgery, (2011) 46, 22-32, d'implanter un ballon par voie endoscopique dans la trachée du fœtus et de remplir ce ballon de fluide afin de bloquer dans les poumons des sécrétions pulmonaires en amont du ballon entraînant une hyperpression qui stimule le développement pulmonaire.

Lorsqu'une telle technique est appliquée, les études montrent une amélioration significative du développement pulmonaire, augmentant notablement les chances de survie du nouveau-né après la naissance.

Pour être efficace, l'implantation d'un ballon dans la trachée du fœtus doit donc obstruer les voies respiratoires naturelles du fœtus. Il est cependant nécessaire de dégonfler le ballon, en réalisant une nouvelle endoscopie ou alors en perçant la paroi du ballon, afin de désobstruer les voies respiratoires naturelles.

Cette opération est réalisée in utero vers 34 semaines d'aménorrhée ou avant en cas de rupture de la poche des eaux ou de début de travail. En effet, le retrait du ballon avant la naissance est crucial pour achever une maturation cellulaire adéquate des poumons, qui augmente les chances de survie néonatale. Le retrait in utero facilite en outre la gestion néonatale, et permet d'envisager dans certains cas un accouchement par voie vaginale.

Des difficultés résultent du fait qu'une telle opération ne peut être faite que par une équipe spécialisée, que cette opération n'est pas toujours techniquement réalisable et qu'elle est associée à une morbi-mortalité périnatale élevée.

Par ailleurs, il existe toujours un risque que l'accouchement se produise avant le dégonflage du ballon. Ceci peut avoir des conséquences dramatiques pour le nouveau-né, si l'équipe en charge de l'accouchement ne parvient pas à retirer le ballon lors du travail, ou rapidement après la délivrance.

Les patientes portant un fœtus ayant un ballon dans la trachée sont ainsi astreintes à rester à proximité ou au sein d'un centre hospitalier apte à effectuer une telle intervention rapidement et de manière la plus sûre.

Ceci est fastidieux et coûteux, dans les cas où la patiente n'habite pas au voisinage d'un tel centre hospitalier.

Un but de l'invention est d'obtenir un ballonnet gonflable facile et pratique à implanter dans une cavité corporelle, notamment dans la trachée d'un fœtus, et qui peut néanmoins être simplement dégonflé lorsque cela est souhaité.

À cet effet, l'invention a pour objet un ballonnet selon la revendication 1.

Le ballonnet selon l'invention peut comprendre l'une ou plusieurs des caractéristiques des revendications 2 à 12, prise(s) isolément ou suivant toute combinaison techniquement possible.

L'invention a également pour objet un nécessaire de traitement d'un patient selon la revendication 13.

Par ailleurs un procédé de vidange d'un ballonnet est ici décrit.

Par ailleurs on décrit ici un procédé d'ouverture d'un orifice de vidange d'un fluide dans un implant, l'implant comportant un organe d'obturation de l'orifice de vidange, propre à libérer l'orifice de vidange sous l'effet d'un champ magnétique pour permettre le passage au moins partiel du fluide présent dans l'implant à travers l'orifice de vidange, le procédé comprenant les étapes suivantes :
- soumission de l'implant à un champ magnétique externe issu d'un appareil de résonance magnétique nucléaire suivant au moins une première direction ;
- déplacement de l'organe d'obturation suivant au moins un axe sous l'effet du champ magnétique externe, pour libérer l'orifice de vidange ;
- passage de fluide à travers l'orifice de vidange.

Le procédé d'ouverture peut comprendre une étape de soumission de l'implant au champ magnétique externe produit par l'appareil de résonance magnétique nucléaire, suivant au moins une deuxième direction, distincte de la première direction.

On décrit également une méthode de traitement chirurgical comprenant les étapes suivantes :
- fourniture d'un nécessaire tel que défini plus haut ;
- amenée, avantageusement par endoscopie, du ballonnet dans une cavité corporelle, à l'aide du dispositif de largage et de gonflage ;
- gonflage du ballonnet dans la cavité corporelle ;
- largage du ballonnet dans la cavité corporelle et retrait du dispositif de gonflage et de largage hors de la cavité corporelle.

La méthode de traitement chirurgical peut comprendre la caractéristique suivante :
- l'étape d'amenée comporte le convoyage du ballonnet dans la cavité amniotique d'une patiente, puis son introduction dans la trachée d'un fœtus présent dans la cavité amniotique.

L'invention sera mieux comprise à la lecture de la description qui va suivre, donnée uniquement à titre d'exemple, et faite en se référant aux dessins annexés, sur lesquels :
- la figure 1 est une vue schématique d'un premier nécessaire de traitement selon l'invention, avant l'implantation du ballonnet dans une cavité corporelle ;
- la figure 2 est un détail illustrant l'orifice de vidange du ballonnet, et l'organe d'obturation de l'orifice dans une position d'obturation, vu depuis l'intérieur du ballonnet ;
- les figures 3 à 6 illustrent les étapes successives d'introduction du ballonnet dans la cavité corporelle ;
- la figure 7 est une vue en perspective du ballonnet gonflé, lors de son largage ;
- la figure 8 est une vue analogue à la figure 7, lors du déplacement de l'organe d'obturation du ballonnet pour libérer l'orifice de vidange, sous l'effet d'un champ magnétique extérieur à la patiente ;
- la figure 9 est une vue en perspective de trois-quarts face d'un deuxième ballonnet selon l'invention, avant son gonflage ;
- la figure 10 est une vue prise en coupe suivant un plan axial médian du ballonnet de la figure 9, lors de son gonflage ;
- la figure 11 est une vue analogue à la figure 9, après gonflage du ballonnet ;
- la figure 12 est une vue analogue à la figure 10, lors du dégonflage ;
- la figure 13 est une vue analogue à la figure 12 d'un troisième ballonnet selon l'invention ;
- la figure 14 est une vue d'un détail d'une variante de ballonnet selon l'invention.

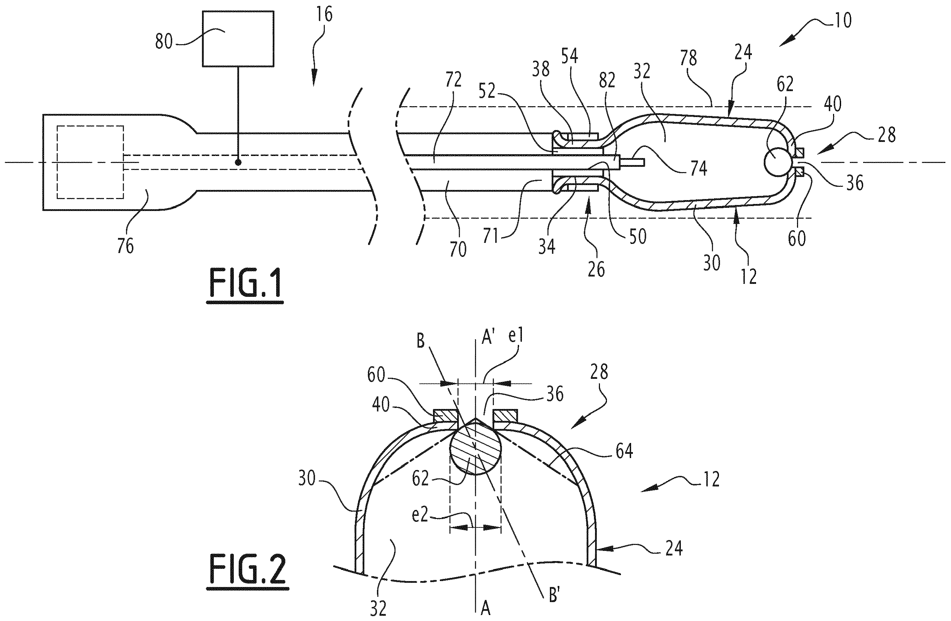

Un premier nécessaire de traitement 10 selon l'invention est illustré schématiquement sur la figure 1.

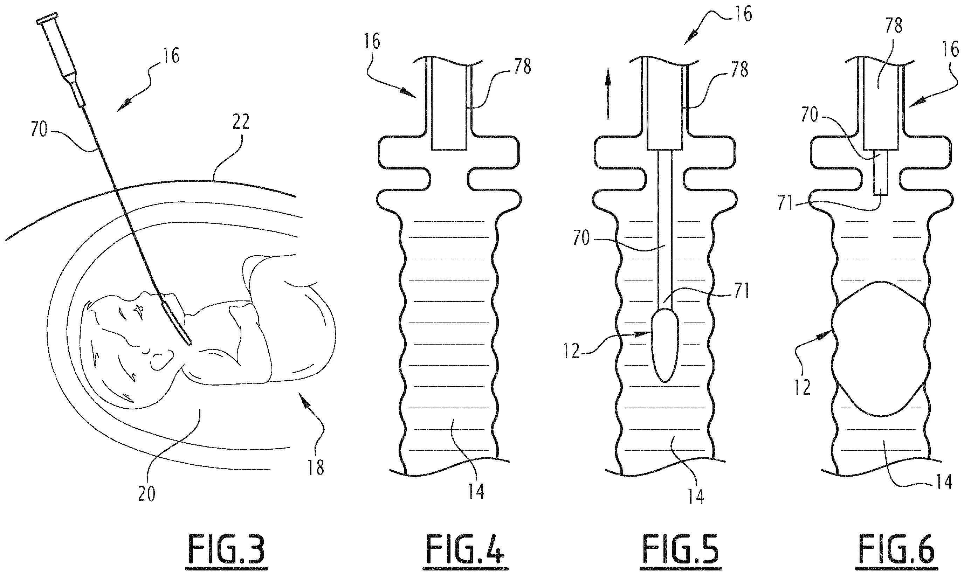

Le nécessaire de traitement 10 comporte un ballonnet gonflable 12 selon l'invention, destiné à être implanté dans une cavité corporelle 14, visible sur les figures 4 à 6. Le nécessaire 10 comporte en outre un dispositif 16 de gonflage et de largage du ballonnet gonflable 12 dans la cavité 14, illustré en particulier sur les figures 1 et 3.

Dans l'application particulière représentée sur les figures 3 à 6, la cavité 14 est la trachée d'un fœtus 18, présent dans la cavité amniotique 20 d'une patiente 22. Le fœtus 18 souffre par exemple d'une hernie diaphragmatique congénitale.

En référence aux figures 1 et 2, le ballon 12 comporte une poche 24 gonflable par un fluide, une vanne 26 de remplissage de la poche 24, et selon l'invention, une vanne 28 de vidange, libérable sous l'effet d'un champ magnétique extérieur à la patiente 22. La vanne de vidange 28 est ici distincte de la vanne de remplissage 26.

La poche 24 est formée d'une paroi étanche 30 déformable au toucher, délimitant un espace intérieur 32 de volume variable en fonction de la quantité de fluide qu'il contient.

La paroi étanche 30 est par exemple réalisée à partir d'un matériau polymère tel que du silicone, du latex, ou d'un caoutchouc ou tel que le polyisoprène.

L'épaisseur de la paroi étanche 30 est inférieure à 1 mm et est généralement comprise entre 0,1 mm et 0,5 mm.

La poche 24 présente généralement une forme allongée le long d'un axe A-A', visible sur la figure 2.

En référence à la figure 1, la poche 24 définit un orifice 34 de remplissage de l'espace intérieur 32, obturé sélectivement par la vanne de remplissage 26 et un orifice 36 de vidange, distinct de l'orifice de remplissage 34, obturé de manière sélective par la vanne de vidange 28.

Dans cet exemple, l'orifice de remplissage 34 est situé à une extrémité proximale de la poche 24, prise le long de l'axe A-A'. L'orifice 34 est délimité à sa périphérie par un manchon 38 de montage de la vanne 26 faisant saillie le long de l'axe A-A' par rapport à la paroi 30. Le manchon 38 est d'un seul tenant avec la paroi 30

L'orifice de vidange 36 est ménagé de manière traversante dans une région périphérique distale 40 de la paroi 30, située à l'opposé de l'orifice de remplissage 34 dans cet exemple.

L'étendue transversale de l'orifice de vidange 36 est avantageusement inférieure à 1,5 mm et est comprise par exemple entre 1 mm et 1,5 mm.

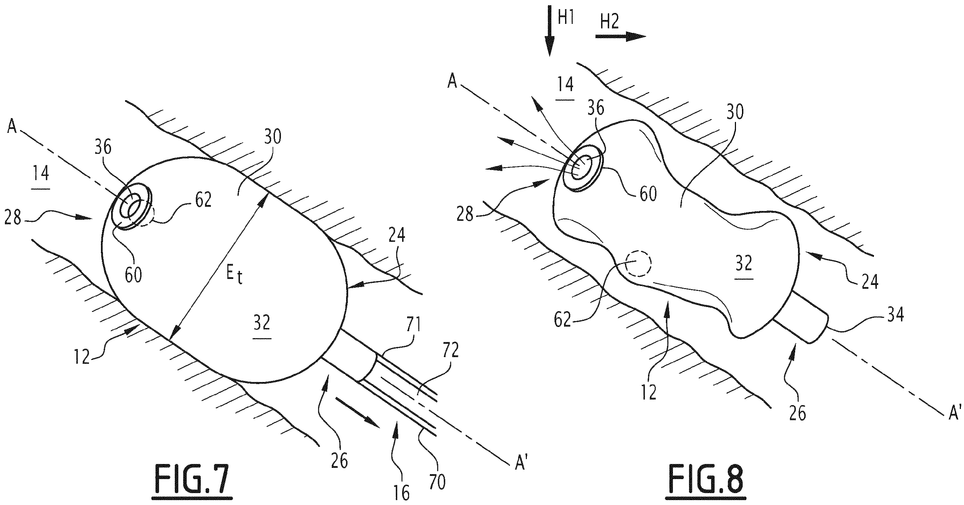

L'espace intérieur 32 de la poche 24 est propre à être rempli par un fluide, de préférence par un liquide, à travers la vanne de remplissage 26, pour passer la poche 24 d'une configuration dégonflée, contractée radialement (visible sur la figure 1 ou sur la figure 5) à une configuration gonflée, dilatée radialement (visible sur la figure 7).

Dans la configuration dégonflée, la poche 24 présente avantageusement une étendue transversale maximale et, prise perpendiculairement à l'axe A-A', avantageusement inférieure à 1,5 mm et comprise généralement entre 1 mm et 1,5 mm.

La longueur de la poche 24, prise le long de l'axe A-A', est avantageusement comprise entre 5 mm et 10 mm.

Le volume de l'espace intérieur 32 est alors compris avantageusement entre 3 mm³ et 10 mm³.

Dans la configuration gonflée, la poche 24 présente avantageusement une étendue transversale maximale Et, prise perpendiculairement à l'axe A-A', avantageusement inférieure à 10 mm et comprise par exemple entre 5 mm et 9 mm.

La longueur de la poche 24 dans la configuration gonflée, prise le long de l'axe A-A' est supérieure à la longueur de la poche 24 dans la configuration dégonflée. Cette longueur est avantageusement comprise entre 15 mm et 25 mm.

Le volume de l'espace intérieur 32 est alors compris avantageusement entre 250 mm³ et 1600 mm³.

Le fluide de gonflage de la poche est par exemple un liquide, notamment un liquide physiologique. Ce liquide contient éventuellement un agent de contraste propre à être visible par radiographie.

En référence à la figure 1, la vanne de remplissage 26 est normalement fermée. Elle définit une lumière centrale 50 d'injection de fluide dans l'espace intérieur 32.

Dans cet exemple, la vanne de remplissage 26 fait saillie axialement par rapport à la paroi 30. Elle est montée autour du manchon 38.

Elle comporte, à l'intérieur du manchon 38, un joint annulaire déformable 52 définissant la lumière centrale 50, et une bague périphérique 54 montée autour du manchon 38 pour enserrer le manchon 38 et le joint 52.

Le joint annulaire 52 est disposé dans l'orifice de remplissage 34. Il est déformable radialement par compression, pour permettre l'introduction d'un élément de remplissage de l'espace intérieur 32. Il est propre à revenir spontanément vers une configuration d'obturation de l'orifice de remplissage 34.

En référence à la figure 2, la vanne de vidange 28 comporte un siège 60 fixé sur la paroi étanche 30, sur la région périphérique 40 autour de l'orifice de vidange 36, et un organe d'obturation 62 de l'orifice de vidange 36, déplaçable suivant au moins deux axes A-A', B-B' distincts, sous l'effet d'un champ magnétique extérieur à la patiente 22.

Dans cet exemple, le siège 60 est rapporté sur la région périphérique 40, à l'extérieur de la paroi étanche 30. Il est par exemple collé sur la région périphérique 40.

Le siège 60 présente ici une forme d'anneau entourant l'orifice de vidange 36.

Le siège 60 est réalisé à l'aide d'un matériau métallique ferromagnétique, propre à être aimanté par un aimant permanent.

L'organe d'obturation 62 est disposé ici dans l'espace intérieur 32. Comme visible sur la figure 2, il présente une étendue transversale maximale e2 supérieure à l'étendue transversale maximale e1 de l'orifice de vidange 36. Dans cet exemple, l'organe d'obturation 62 est une bille.

L'organe d'obturation 62 présente une aimantation permanente. Il est ainsi propre à coopérer magnétiquement avec le siège 60 pour être maintenu dans une position d'obturation de l'orifice de vidange 36, dans laquelle il est appliqué contre la région périphérique 40 en regard du siège 60.

Dans cette position, l'organe d'obturation 62 obture totalement l'orifice 36, et empêche le passage de fluide depuis le volume intérieur 32 vers l'extérieur de la poche 24.

L'étanchéité autour de l'orifice de vidange 36 est renforcée par la présence de la région périphérique 40, sur laquelle s'appuie l'organe d'obturation 62, qui forme une couche intermédiaire déformable.

Sous l'effet d'un champ magnétique extérieur, propre à engendrer une force d'attraction de l'organe d'obturation 62 supérieure à la force de coopération entre l'organe d'obturation 62 et le siège 60, l'organe d'obturation 62 est propre à se déplacer à l'écart de l'orifice de vidange 36 dans l'espace intérieur 32 suivant au moins deux axes A-A', B-B'.

L'intensité du champ magnétique propre à libérer l'organe d'obturation 62 est par exemple supérieure à 0,1 T et est notamment comprise entre 0,5 T et 2 T.

En pratique, lorsqu'il se détache de la position d'obturation, l'organe d'obturation 62 est propre à se déplacer librement suivant une multitude d'axes dans un cône 64 centré sur l'axe A-A' de l'orifice de vidange 36, au niveau de l'orifice 36. Le cône 64 présente un angle d'ouverture vers l'espace intérieur 32 supérieur à 30°, de préférence supérieur à 90°, et avantageusement égal à 180°.

Aucun moyen mécanique de retenue ne raccorde l'organe d'obturation 62 à la poche gonflable 24 et/ou au siège 60.

Une fois détachée de la position d'obturation, l'organe d'obturation 62 est propre à atteindre au moins une position de libération de l'orifice 36, cette position dépendant de l'orientation de la patiente 22, et de celle du champ magnétique extérieur.

En particulier, l'organe d'obturation 62 est propre à occuper une pluralité de positions de libération distinctes, dans l'espace intérieur 32, après avoir quitté la position d'obturation, dont une est illustrée sur la figure 8.

En référence aux figures 1 et 3, le dispositif 16 de gonflage et de largage du ballonnet 12 comporte un tuteur souple 70 portant à son extrémité distale 71 le ballonnet 12, un tube de gonflage 72 disposé dans le tuteur 70, et avantageusement, une tige mandrin 74 disposée dans le tube de gonflage 72 pour le rigidifier.

Le dispositif 16 comporte en outre un embout proximal 76, pour sa manipulation par un praticien, et avantageusement, une gaine amovible 78 de protection du ballonnet 12.

Le tuteur 70 s'étend entre l'embout proximal 76 et l'extrémité distale 71. Il est propre à être déformé pour être introduit dans la patiente 22 avantageusement par voie endoscopique, et atteindre la cavité 14.

Le tube de gonflage 72 s'étend à travers le tuteur 70. Il est raccordé en amont à un réservoir 80 d'injection de fluide.

Le tube de gonflage 72 présente une partie distale 82 qui fait saillie par rapport à l'extrémité distale 71 du tuteur 70 pour être introduite dans la lumière centrale 50 de la vanne de remplissage 26.

La gaine amovible 78 est propre à couvrir le ballonnet 12 lors de son introduction jusqu'à la cavité 14. Elle est mobile longitudinalement autour du tuteur 70 pour découvrir le ballonnet 12, à son point d'implantation dans la cavité 14.

Le fonctionnement du nécessaire de traitement 10 dans le cadre d'une implantation dans une cavité corporelle 14 va maintenant être décrit.

Cette implantation est par exemple réalisée dans la trachée d'un fœtus 18 présent dans la cavité amniotique 20 d'une patiente 22.

Initialement, le ballonnet 12 est monté à l'extrémité distale 71 du tuteur 70 du dispositif de gonflage et de largage 16. La partie distale 82 du tube de gonflage 72 est introduite dans la vanne de remplissage 26, par déformation radiale du joint annulaire 52.

L'organe d'obturation 62 est plaqué en regard du siège 60 contre la région périphérique 40. Il occupe sa position d'obturation de l'orifice 36.

La poche 24 occupe alors sa configuration dégonflée, d'étendue radiale minimale, visible sur les figures 1 et 5.

Le dispositif 16, muni du ballonnet 12 à son extrémité est alors introduit dans la patiente 22, par voie endoscopique. Dans l'exemple représenté sur la figure 3, le praticien l'introduit dans la cavité amniotique 20, puis l'amène à travers les voies respiratoires du fœtus 18 jusqu'à la trachée, en passant à travers les cordes vocales (figure 4).

Une fois l'extrémité distale 71 dans la cavité 14, le praticien extrait le ballonnet 12 hors de la gaine 78, en tirant la gaine 78 vers l'embout proximal 76, comme visible sur la figure 5.

La poche gonflable 24 occupe toujours sa configuration dégonflée.

Le praticien injecte alors du fluide de gonflage dans l'espace intérieur 32 à travers le tube de gonflage 72 introduit dans la vanne de remplissage 26. La poche 24 se dilate radialement pour atteindre sa configuration gonflée, en appui sur la paroi délimitant la cavité 14, comme visible sur la figure 6.

Puis, le praticien détache le ballonnet 12 du dispositif de gonflage et de largage 16, en extrayant le tube de gonflage 72 hors de la vanne de remplissage 26. La vanne de remplissage 26 se referme par dilatation radiale du joint annulaire 52.

Le praticien retire alors le dispositif 16 de la patiente 22.

Durant le gonflage, et après celui-ci, l'organe d'obturation 62 reste dans sa position d'obturation. Le fluide de gonflage reste donc confiné dans le volume intérieur 32.

La cavité 14 est alors obturée. Dans le cas d'un fœtus 18 souffrant d'hernie diaphragmatique congénitale, le développement pulmonaire du fœtus est amélioré, grâce à la présence du ballonnet 12 gonflé dans la trachée.

Lorsque le ballonnet 12 doit être retiré, la patiente est soumise à un champ magnétique extérieur de forte intensité, par exemple d'intensité supérieure à 0,1 T.

Ce champ magnétique est par exemple produit par un appareil d'imagerie par résonance magnétique. Selon les circonstances, la patiente se positionne dans l'appareil lors d'une acquisition d'image ou de préférence sans acquisition d'images dans l'appareil. Préférentiellement, la patiente n'a pas besoin de se positionner dans l'appareil, le champ de fuite de l'appareil allumé ou à l'entrée du tunnel est avantageusement suffisant pour produire un champ magnétique adéquat pour libérer l'organe d'obturation 62, auquel cas la patiente se tiend simplement debout devant la machine.

De préférence, comme illustré par la figure 8, l'orientation relative entre le champ magnétique et la patiente 22 est modifié, par exemple en déplaçant la patiente, pour que le champ magnétique soit appliqué suivant au moins deux axes H1 et H2 distincts, comme illustré par la figure 8.

Le champ magnétique extérieur engendre une force d'attraction sur l'organe d'obturation 62 qui surmonte la force de coopération entre l'organe d'obturation 62 et le siège 60.

Sous l'effet du champ magnétique extérieur, l'organe d'obturation 62 se déplace à l'écart de l'orifice de vidange 36, jusqu'à une position de libération de l'orifice 36, représentée par exemple sur la figure 8.

Au moins une partie du fluide présent dans l'espace intérieur 32 s'écoule alors depuis l'espace intérieur 32 vers l'extérieur, à travers l'orifice de vidange 36, provoquant le dégonflage rapide du ballonnet 12.

Les voies respiratoires du fœtus sont alors à nouveau dégagées. Le ballonnet 12 est alors apte à être expulsé hors du fœtus par la libération du fluide pulmonaire en surpression, ou est retiré quelques jours après la naissance.

La libération de l'organe d'obturation 62 est immédiate et très facile à réaliser. Aucune liaison mécanique n'existant entre d'une part, l'organe d'obturation 62 et d'autre part, la poche gonflable 24 ou le siège 60, cette libération est très fiable et ne dépend pas d'un mécanisme mécanique ou électrique. Au contraire, le dégagement de l'orifice de vidange 36 est exclusivement provoqué par le champ magnétique extérieur appliqué, combiné aux forces de gravité s'appliquant sur l'organe d'obturation 62.

Cette libération est en outre non invasive pour la patiente 22, puisqu'elle peut être réalisée à distance, sans avoir à inciser la patiente 22, voire à pénétrer dans la cavité amniotique 20.

La vanne de vidange 28 ainsi obtenue est peu coûteuse à réaliser, tout en assurant un fonctionnement adéquat, quelles que soient les circonstances.

Les risques pour le fœtus 18 sont donc totalement écartés, puisque le retrait du ballonnet 12 est facilité par son dégonflage immédiat, et son expulsion hors de la trachée, au moment souhaité et sans procédure invasive.

La patiente 22 bénéficie d'un traitement adéquat, assurant le bon développement de son fœtus 18, sans nécessairement être maintenue au voisinage ou au sein d'un centre hospitalier spécialisé, ce qui limite les coûts en maintenant la qualité de soins.

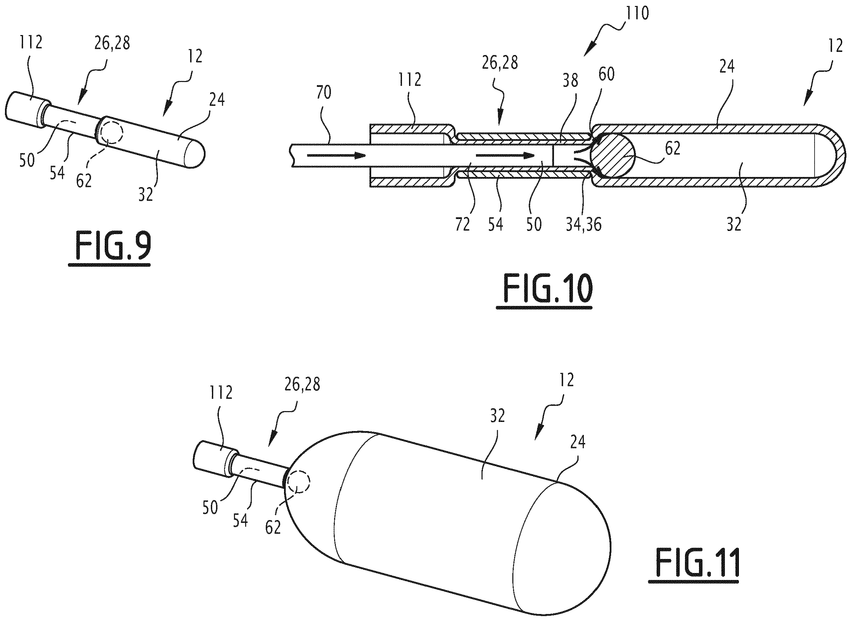

Un deuxième nécessaire de traitement 110 selon l'invention est illustré par les figures 9 à 13.

Le ballonnet 12 du deuxième nécessaire 110 est formé de manière similaire au ballonnet 12 du premier nécessaire 10. En particulier, les dimensions du ballonnet 12 du deuxième nécessaire 110 sont analogues à celles du ballonnet 12 du premier nécessaire 10.

Toutefois, à la différence du premier nécessaire 10, le ballonnet 12 comporte une vanne unique formant à la fois une vanne de remplissage 26 et une vanne de vidange 28.

L'orifice de vidange 36 est constitué par l'orifice de remplissage 34 à l'extrémité de la vanne 26, 28. La poche 24 est donc munie d'un orifice 34, 36 unique permettant le remplissage de l'espace intérieur 32 en fluide et la vidange du fluide contenu dans l'espace intérieur 32.

L'organe d'obturation 62 obture par défaut la vanne de remplissage 26. Ainsi, la bague périphérique 54 délimitant la vanne de remplissage 26 est dépourvue de joint annulaire. Elle définit donc une lumière centrale 50 dégagée en permanence.

Comme pour le ballonnet 12 du premier nécessaire 10, la poche 24 se prolonge par un manchon 38 inséré dans la bague périphérique 54 et délimitant la périphérie de la lumière centrale 50.

Dans cet exemple, le manchon 38 se prolonge au-delà de la bague 54, à l'opposé de la poche 24, par un embout annulaire 112 s'ouvrant vers l'extérieur.

La bague périphérique 54 est ici en matériau métallique ferromagnétique. Elle définit le siège 60, sur lequel s'appuie l'organe d'obturation 62 dans la position d'obturation, sur la région périphérique 40.

L'organe d'obturation 62 présente ici une aimantation permanente. Il s'applique donc au repos sur le siège 60, dans la position d'obturation.

Le fonctionnement du deuxième nécessaire 110 selon l'invention est analogue à celui du premier nécessaire 10.

Toutefois, à la différence du premier nécessaire 10, le tube de gonflage 72 est introduit dans la bague périphérique 54 de la vanne de remplissage 26, sans ouvrir l'orifice de remplissage 34, puisque l'organe d'obturation 62 reste appliqué sur le siège 60.

Comme illustré par la figure 10, la force hydraulique générée par la pression du fluide injecté par le tube de gonflage 72, pousse l'organe d'obturation 62 à l'écart du siège 60 et surmonte au moins partiellement la force magnétique retenant l'organe d'obturation 62 sur le siège 60.

Un interstice se forme alors entre le siège 60 et l'organe d'obturation 62 permettant l'entrée de fluide dans l'espace intérieur 32 et le gonflage de la poche 24.

Lorsque le gonflage s'achève, l'organe d'obturation 62 reprend sa position d'obturation sous l'effet de la force magnétique. L'espace intérieur 32 de fluide est obturé de manière étanche, comme l'illustre la figure 11. Une pression induite, par exemple supérieure à 0,5 bars relatifs, et notamment de l'ordre de 1 bar relatifs subsiste dans l'espace intérieur 32 du ballonnet 12.

Cette pression est nécessaire au démarrage du gonflage (amorçage de l'élasticité du ballonnet 12). Cette pression baisse dans la deuxième phase du gonflage. La pression induite dépend de l'élasticité du matériau, de l'épaisseur de paroi, de la longueur initiale du ballonnet, etc. Elle favorise l'étanchéité de la vanne.

Pour dégonfler le ballonnet 12, la patiente est soumise à un champ magnétique extérieur de forte intensité, comme décrit précédemment.

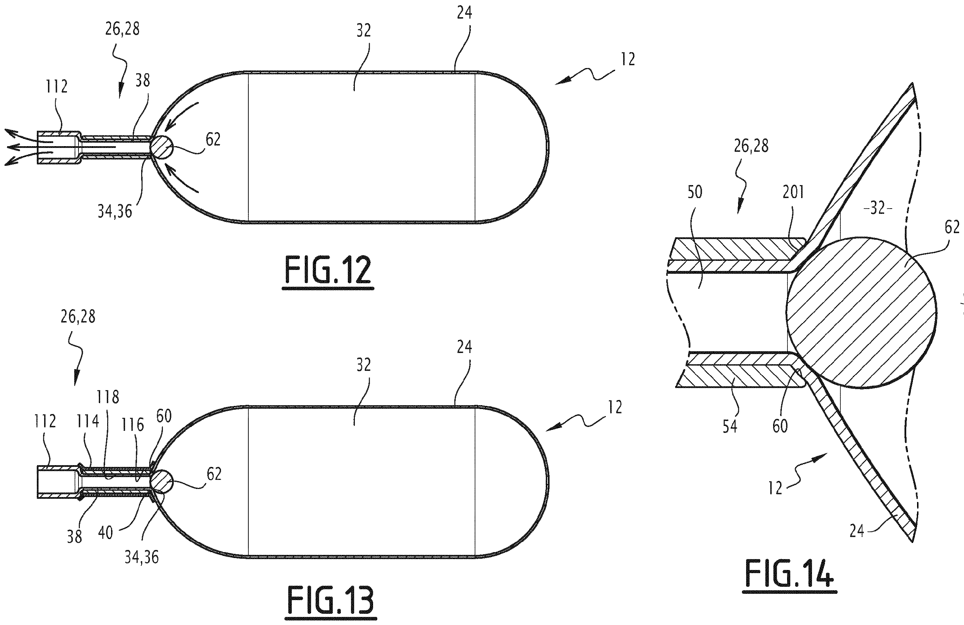

Comme visible sur la figure 12, ceci engendre la libération de l'organe d'obturation 62 au moins temporairement à l'écart du siège 60 et le passage de fluide autour de l'organe d'obturation 62 vers la lumière centrale 50 pour dégonfler la poche 24.

Le déplacement de l'organe d'obturation 62 à l'écart du siège 60 est avantageusement très limité, par exemple limitée à quelques centièmes, voir quelques dixièmes de millimètres.

L'organe d'obturation 62 est cependant déplaçable suivant au moins deux axes distincts par rapport à la poche 24, comme pour le ballonnet 12 du premier nécessaire 10, ce qui ne nécessite pas de contrôler l'orientation du champ magnétique suivant une direction spécifique pour libérer l'organe d'obturation 62.

Avantageusement, pour chacun des ballonnets 12 décrits précédemment, l'organe d'obturation 62 et/ou la bague périphérique 54 sont munis d'un revêtement biocompatible 114.

Ce revêtement est par exemple un revêtement en titane, en carbone, en polymère fluoré (notamment en polytétrafluoroéthylène), ou/et en parylène. En variante, le revêtement est un film, notamment un film polymère, par exemple en polyisoprène, ou en polyuréthane.

Dans l'exemple représenté sur la figure 13, au moins une surface périphérique extérieure 116 de la bague 54 est munie du revêtement biocompatible. Avantageusement, une surface périphérique intérieure 118 de la bague 54 et la surface extérieure de l'organe d'obturation 62 sont également munies de ce revêtement,

Dans encore une autre variante, visible sur la figure 14, la bague 54 présente un chanfrein 201 à son extrémité formant le siège 60 de l'organe d'obturation 62.

L'organe d'obturation 62 est ainsi apte à entrer plus dans le diamètre intérieur de la bague 54. Ceci augmente la surface de contact et donc l'étanchéité entre l'organe d'obturation 62 et la bague 54, prise au niveau du siège 60.

Dans une variante (non représentée), l'organe d'obturation 62 après avoir quitté la position d'obturation n'est pas nécessairement déplaçable librement dans tout l'espace intérieur 32, mais uniquement dans une région limitée de l'espace intérieur 32. Par exemple, un compartiment de réception de l'organe d'obturation 62 est monté dans l'espace intérieur 32 autour de l'orifice de vidange 36.

Dans une autre variante (non représentée), l'organe d'obturation 62 est disposé dans sa position d'obturation à l'extérieur de l'espace intérieur 32. Un compartiment de réception de l'organe d'obturation 62 est monté sur la poche 24, à l'extérieur de celle-ci, autour de l'orifice de vidange 36. Ce compartiment de réception est de préférence ajouré pour permettre l'écoulement du fluide provenant de l'espace intérieur 32.

Dans d'autres variantes, l'organe d'obturation 62 ne présente pas une forme sphérique mais présente une autre forme, par exemple polyédrique.

## Revendications

1. Ballonnet (12) gonflable, destiné à être implanté dans une cavité (14) corporelle, comportant :
- une poche (24) formée d'une paroi étanche (30) délimitant un espace intérieur (32) ;
- une vanne de remplissage (26) de l'espace intérieur (32) par un fluide, propre à être obturée après remplissage de l'espace intérieur (32) ;
la poche (24) délimitant un orifice de vidange (36) de fluide débouchant dans l'espace intérieur (32), le ballonnet (12) comportant un organe d'obturation (62) de l'orifice de vidange (36), l'organe d'obturation (62) étant propre à libérer l'orifice de vidange (36) sous l'effet d'un champ magnétique, pour permettre la vidange au moins partielle du fluide contenu dans l'espace intérieur (32), **caractérisé en ce que** l'organe d'obturation (62) est déplaçable suivant au moins deux axes distincts par rapport à la poche (24).

2. Ballonnet (12) selon la revendication 1, dans lequel l'organe d'obturation (62) est disposé dans l'espace intérieur (32).

3. Ballonnet (12) selon la revendication 2, dans lequel l'organe d'obturation (62) est librement déplaçable dans l'espace intérieur (32) défini par la poche (24) sous l'effet d'un champ magnétique.

4. Ballonnet (12) selon l'une quelconque des revendications précédentes, dans lequel l'organe d'obturation (62) est propre à être maintenu dans une position d'obturation de l'orifice de vidange (36) par aimantation, l'organe d'obturation (62) étant propre à être déplacé à l'écart de l'orifice de vidange (36) sous l'effet d'un champ magnétique propre à vaincre l'aimantation maintenant l'organe d'obturation (62) dans la position d'obturation de l'orifice de vidange (36).

5. Ballonnet (12) selon l'une quelconque des revendications précédentes, comprenant au moins un siège de retenue (60) de l'organe d'obturation (62), disposé au voisinage de l'orifice de vidange (36), l'organe d'obturation (62) coopérant par aimantation avec le siège de retenue (60) dans une position d'obturation de l'orifice de vidange (36), le siège de retenue (60) étant préférentiellement un anneau, rapporté sur la paroi étanche (30) autour de l'orifice de vidange (36).

6. Ballonnet (12) selon la revendication 5, dans lequel le siège de retenue (60) est couvert d'une couche d'un matériau souple, l'organe d'obturation (62) étant disposé en appui sur la couche de matériau souple dans une position d'obturation de l'orifice de vidange (36).

7. Ballonnet (12) selon l'une quelconque des revendications précédentes, dans lequel l'organe d'obturation (62) est une bille.

8. Ballonnet (12) selon l'une quelconque des revendications précédentes, dans lequel l'organe d'obturation (62) présente une aimantation permanente.

9. Ballonnet (12) selon l'une quelconque des revendications précédentes, dans lequel l'organe d'obturation (62) est propre à être déplacé jusqu'à au moins deux positions distinctes de libération de l'orifice de vidange (36) sur deux axes sécants passant par l'orifice de vidange.

10. Ballonnet (12) selon l'une quelconque des revendications précédentes, dans lequel la paroi étanche (30) de la poche (24) est réalisée à partir d'un polymère choisi parmi le silicone, le latex, le polyuréthane, et/ou le polyisoprène.

11. Ballonnet (12) selon l'une quelconque des revendications précédentes, dans lequel l'orifice de vidange (36) est défini par la vanne de remplissage (26), l'organe d'obturation (62) étant propre à fermer la vanne de remplissage (26) après remplissage de l'espace intérieur (32).

12. Ballonnet (12) selon l'une quelconque des revendications précédentes, dans lequel la vanne de remplissage (26) comporte une bague de guidage (54) d'un tube (72) de gonflage du ballonnet (12), la bague de guidage (54) et/ou l'organe d'obturation (62) présentant un revêtement biocompatible (114).

13. Nécessaire (10) de traitement d'un patient, comportant :
- un ballonnet (12) selon l'une quelconque des revendications précédentes ;
- un dispositif (16) de gonflage et de largage du ballonnet (12), comprenant un tuteur (70) de support du ballonnet (12), le ballonnet (12) étant monté de manière libérable sur le tuteur de support (70), et un tube (72) de gonflage du ballonnet (12), propre à être inséré de manière libérable dans la vanne de remplissage (26).

## Patentansprüche

1. Aufblasbarer Ballon (12), der vorgesehen ist, in eine Körperhöhle implantiert zu werden und aufweist:
- eine Blase (24), die aus einer dichten, einen Innenraum (32) begrenzenden Wand (30) gebildet ist;
- ein Ventil (26) zum Füllen des Innenraums (32) mit einem Fluid, wobei das Ventil geeignet ist, nach dem Füllen des Innenraums (32) verschlossen zu werden;
wobei die Blase (24) eine in den Innenraum (32) mündende Auslassöffnung (36) für das Fluid begrenzt und die Blase (12) ein Verschlusselement (62) für die Auslassöffnung (36) aufweist und das Verschlusselement (62) geeignet ist, die Auslassöffnung (36) durch die Wirkung eines Magnetfeldes freizulegen, um zumindest teilweise das Ablassen des in dem Innenraum (32) enthaltenen Fluids zuzulassen, **dadurch gekennzeichnet, dass** das Verschlusselement (62) gemäß mindestens zweier unterschiedlicher Achsen in Bezug auf die Blase (24) bewegbar ist.

2. Ballon (12) nach Anspruch 1, bei dem das Verschlusselement (62) in dem Innenraum (32) angeordnet ist.

3. Ballon (12) nach Anspruch 2, bei dem das Verschlusselement (62) in dem von der Blase (24) definierten Innenraum (32) durch die Wirkung eines Magnetfeldes frei bewegbar ist.

4. Ballon (12) nach einem beliebigen der vorhergehenden Ansprüche, bei dem das Verschlusselement (62) geeignet ist, durch Magnetisierung in einer Verschlussposition der Auslassöffnung (36) gehalten zu werden und das Verschlusselement (62) geeignet ist, durch die Wirkung eines Magnetfeldes von der Auslassöffnung wegbewegt zu werden, wobei das Magnetfeld geeignet ist, die das Verschlusselement (62) in der Verschlussposition der Auslassöffnung (36) haltenden Magnetisierung zu überwinden.

5. Ballon (12) nach einem beliebigen der vorhergehenden Ansprüche, der mindestens einen Rückhaltesitz (60) für das Verschlusselement (62) umfasst, der benachbart zur Auslassöffnung (36) angeordnet ist, wobei das Verschlusselement (62) durch Magnetisierung mit dem Rückhaltesitz in der Verschlussposition der Auslassöffnung (36) zusammenarbeitet und der Rückhaltesitz (60) vorzugsweise ein Ring ist, der an die dichte Wand (30) um die Auslassöffnung (36) herum angesetzt ist.

6. Ballon (12) nach Anspruch 5, bei dem der Rückhaltesitz (60) mit einer Schicht aus weichem Material bedeckt ist, wobei das Verschlusselement (62) in einer Verschlussposition der Auslassöffnung (36) in Anlage an die Schicht des weichen Materials angeordnet ist.

7. Ballon (12) nach einem beliebigen der vorhergehenden Ansprüche, bei dem das Verschlusselement (62) eine Kugel ist.

8. Ballon (12) nach einem beliebigen der vorhergehenden Ansprüche, bei dem das Verschlusselement (62) eine Permanentmagnetisierung aufweist.

9. Ballon (12) nach einem beliebigen der vorhergehenden Ansprüche, bei dem das Verschlusselement (62) geeignet ist, in mindestens zwei unterschiedliche Freigabepositionen der Verschlussöffnung (36) auf zwei sich schneidenden und durch die Auslassöffnung gehenden Achsen bewegt zu werden.

10. Ballon (12) nach einem beliebigen der vorhergehenden Ansprüche, bei dem die dichte Wand (30) der Blase (24) aus einem Polymer hergestellt ist, das ausgewählt ist aus Silikon, Latex, Polyurethan und/oder Polyisopren.

11. Ballon (12) nach einem beliebigen der vorhergehenden Ansprüche, bei dem die Auslassöffnung (36) durch das Füllventil (26) definiert ist, wobei das Verschlusselement (62) geeignet ist, das Füllventil (26) nach dem Füllen des Innenraums (32) zu verschließen.

12. Ballon (12) nach einem beliebigen der vorhergehenden Ansprüche, bei dem das Füllventil (26) einen Führungsring (54) für ein Rohr (72) zum Aufblasen des Ballons (12) aufweist, wobei der Führungsring (54) und/oder das Verschlusselement (62) eine biokompatible Beschichtung (114) aufweisen.

13. Necessaire (10) zum Behandeln eines Patienten, aufweisend:
- einen Ballon (12) nach einem beliebigen der vorhergehenden Ansprüche;
- eine Vorrichtung (16) zum Aufblasen und Vergrößern des Ballons (12), die eine Stütze (70) zum Lagern des Ballons (12), wobei der Ballon (12) lösbar auf der Lagerstütze (70) montiert ist, und ein Rohr (72) zum Aufblasen des Ballons (12), das geeignet ist, entfernbar in das Füllventil (26) eingesetzt zu werden, umfasst.

## Claims

1. Inflatable balloon (12), designed to be implanted in a body cavity (14), comprising:
- a pouch (24) formed of a sealed wall (30) delimiting an internal space (32);
- a valve (26) for filling the internal space (32) with a fluid, capable of being occluded after filling the internal space (32);
the pouch (24) delimiting a fluid-draining orifice (36) opening into the internal space (32), the balloon (12) comprising an element (62) that occludes the draining orifice (36), the occluding element (62) being capable of releasing the draining orifice (36) under the effect of a magnetic field, so as to enable the at least partial drainage of the fluid contained in the internal space (32), **characterized in that** the occluding element (62) is movable along at least two distinct axes in relation to the pouch (24).

2. Balloon (12) according to claim 1, wherein the occluding element (62) is arranged in the internal space (32).

3. Balloon (12) according to claim 2, wherein the occluding element (62) is freely movable in the internal space (32) defined by the pouch (24) under the effect of a magnetic field.

4. Balloon (12) according to any of the preceding claims, wherein the occluding element (62) is capable of being held by magnetization in a position of occluding the draining orifice (36), the occluding element (62) being capable of being moved away from the draining orifice (36) under the effect of a magnetic field capable of overcoming the magnetization holding the occluding element (62) in the position of occluding the draining orifice (36).

5. Balloon (12) according to any of the preceding claims, comprising at least one seat (60) to retain the occluding element (62), arranged near the draining orifice (36), the occluding element (62) cooperating by magnetization with the retaining seat (60) in a position that occludes the draining orifice (36), the retaining seat (60) preferably being a ring mounted on the sealed wall (30) around the draining orifice (36).

6. Balloon (12) according to claim 5, wherein the retaining seat (60) is coated with a layer of flexible material, the occluding element (62) being arranged bearing on the layer of flexible material in a position that occludes the draining orifice (36).

7. Balloon (12) according to any of the preceding claims, wherein the occluding element (62) is a ball.

8. Balloon (12) according to any of the preceding claims, wherein the occluding element (62) is permanently magnetized.

9. Balloon (12) according to any of the preceding claims, wherein the occluding element (62) is capable of being moved into at least two distinct positions of releasing the draining orifice (36) on two intersecting axes passing through the draining orifice.

10. Balloon (12) according to any of the preceding claims, wherein the sealed wall (30) of the pouch (24) is made of a polymer chosen from silicone, latex, polyurethane and/or polyisoprene.

11. Balloon (12) according to any of the preceding claims, wherein the draining orifice (36) is defined by the filling valve (26), the occluding element (62) being capable of closing the filling valve (26) after the internal space (32) has been filled.

12. Balloon (12) according to any of the preceding claims, wherein the filling valve (26) comprises a ring (54) to guide a tube (72) for inflating the balloon (12), the guiding ring (54) and/or the occluding element (62) having a biocompatible coating (114).

13. Patient treatment kit (10) comprising:
- a balloon (12) according to any of the preceding claims;
- a balloon (12) inflation and deployment device (16), comprising a balloon (12) support guide (70), the balloon (12) being mounted in a releasable manner on the support guide (70), and a balloon (12) inflation tube (72), capable of being inserted in a releasable manner into the filling valve (26).
